# EUROPEAN PATENT APPLICATION

(11) **EP 2 554 203 A1**
(43) Date of publication of application: **06.02.2013**
(21) Application number: 12005638.7
(22) Date of filing: 02.08.2012
(51) Int. Cl.: A61M 5/31, A61M 39/20

(54) **Plastic-made syringe nozzle cap**

(30) Priority: 05.08.2011 JP 2011171674
(71) Applicant: Daikyo Seiko, LTD., Tokyo 131-0031 (JP)
(72) Inventor: Kawamura, Hideaki, Tokyo 131-0031 (JP)
(74) Representative: Blodig, Wolfgang

(57) **Abstract**

A plastic-made nozzle cap (1) for a plastic syringe includes a leg section (11), a cavity top wall portion (14), and a finger knob (12) for the attachment/detachment of the nozzle cap. The leg section is structured for connection with a female Luer lock fitting (24) formed face-to-face with an outer circumferential wall (23) of a syringe nozzle (21). The cavity top wall portion is provided with a curved surface having a downwardly-convex, radius of curvature ranging from 0.6 to 12.0 mm for being brought into contact with a tip (22) of the syringe nozzle to maintain sealing performance. The finger knob is arranged on an upper side of the leg section, and extends above the female Luer lock fitting in a state that a syringe barrel (2) and the nozzle cap have been connected together. (FIG. 4)

## Description

### Field of the Invention

This invention relates to a plastic-made nozzle cap for a plastic syringe useful with a medicine, and specifically, to a plastic-made nozzle cap that can be firmly connected to a female Luer lock fitting of such a syringe and can also maintain high sealing performance.

### Background of the Invention

Prefilled syringes useful as combined container-syringe units for medicines have been finding an ever-increasing demand for their convenience in recent years. A prefilled syringe is generally constructed of a substantially cylindrical syringe barrel, a substantially cylindrical piston, a plunger rod, and a substantially cylindrical nozzle cap. The syringe barrel is provided at an end thereof with a nozzle formed for the attachment of an injection needle, and is also provided at an opposite end thereof with an open end. The piston can seal up the open end of the syringe barrel, and can slide in the syringe barrel. The plunger rod is connected or connectable to the piston to enable sliding operation of the piston. The nozzle cap is closed at an end thereof to seal up the nozzle of the syringe barrel. This prefilled syringe stores a medical solution in an internal chamber of the syringe barrel as defined by combining the piston and nozzle cap with the syringe barrel.

A prefilled syringe is needed to maintain its sealing performance until the medical solution as its contents is used. For a medicine, a validity term of from a few years to 5 years or so is often set. The sealing performance, therefore, needs to remain during at least the validity term. On the other hand, the storage and shipping conditions for medicines vary widely so that these medicines include those to be exposed to high temperatures, those to be stored at extremely low temperatures, and those to be placed under elevated pressure or reduced pressure. Under such conditions, the constituent material of a prefilled syringe, to say nothing of its medicine itself, cannot avoid a volumetric expansion or shrinkage, and the rate of expansion or shrinkage differs depending on the material. There is, accordingly, an outstanding requirement for prefilled syringes that can maintain sealing performance even under such conditions. For allowing a prefilled syringe to maintain sealing performance, it is necessary that the piston and nozzle cap stably remain in close contact with the syringe barrel under any conditions. As opposed to the piston slidably inserted in the syringe barrel, the nozzle cap has such a shape as covering the nozzle of the syringe barrel from the outside, and is a component that is more difficult to maintain sealing performance than the piston. There is, accordingly, an outstanding desire for providing a nozzle cap with improved sealing performance in a prefilled syringe.

In nozzle caps, rubber-based materials have heretofore been used as their molding materials. For example, WO-A-2008-59863 discloses butyl rubber and chlorinated butyl rubber as constituent materials for nozzle caps. JP-A-2002-153539 discloses that the Lure plugs described therein are made of silicone rubber. Like the end caps disclosed in JP-A-2007-507308, however, nozzle caps made of resin materials have been proposed and put in practical use for excellent workability in recent years.

From the standpoint of providing still more improved sealing performance, however, the above-described nozzle caps made of the rubber-based materials are excellent in sealing performance owing to the rubber elasticity of the rubber-based materials, but on the other hand, are accompanied by a problem that their connection with the associated syringe nozzles has to also rely upon rubber elasticity and frictional resistance. With the above-described nozzle caps made of the conventional resin materials, on the other hand, the connection with the associated syringe nozzles can be assured by the threaded engagement between the resin materials themselves. Nonetheless, the addition of a thermoplastic elastomer as much as from 20 to 50 wt% is needed to impart sufficient elasticity for the assurance of flexibility, in other words, the sealing performance for the nozzle caps, so that a new problem has arisen as will be described hereinafter. The use of a resin material with such a large amount of an elastomer contained therein may raise another problem in that dissolved matter from the elastomer cannot be avoided completely. If the sealing performance has to be maintained for a longer term, the nozzle cap remains to be subjected to stress during that term so that its material itself is known to undergo plastic deformation, that is, so-called stress relaxation. In this case, the sealing performance is impaired. Accordingly, there is also a room for further improvements with respect to the above-described nozzle caps made of the conventional resin materials.

### Disclosure of the Invention

An object of the present invention is to solve these problems, and to provide a plastic-made nozzle cap for a plastic syringe useful with a medicine, which despite being made of a plastic, can be firmly connected to a female Luer lock fitting of the plastic syringe and is also excellent in durability so that high sealing performance can be maintained even when continuously used over a validity term of a medicine.

The above-described object can be achieved by the present invention to be described hereinafter. Described specifically, to solve the above-described problems, the present invention provides a plastic-made nozzle cap for a plastic syringe useful with a medicine, characterized by comprising a leg section structured for connection with a female Luer lock fitting formed face-to-face with an outer circumferential wall of a nozzle of the syringe, a cavity top wall portion provided with a downwardly-convex, curved surface having a radius of curvature ranging from 0.6 to 12. 0 mm such that the cavity top wall portion can be brought into contact with a tip of the nozzle of the syringe to maintain sealing performance, and a finger knob for attachment/detachment of the nozzle cap, said finger knob being arranged on an upper side of the leg section such that the finger knob extends above the female Luer lock fitting in a state that the syringe and the nozzle cap have been connected together.

Preferably, the above-described plastic-made nozzle cap may further comprise threads or a ridge arranged on an outer circumference of the leg section for threaded engagement with the female Luer lock fitting of the syringe. The above-described plastic-made nozzle cap may also further comprise a guide formed for the nozzle on an end part of a cavity side wall, said end part being located adjacent the cavity top wall portion. The guide may comprise, for example, a ring-shaped guide, or plural semicircular column-shaped protrusions formed at positions where the end part of the cavity side wall is divided along an entire circumference thereof into from 3 to 5 equal sections. The plastic-made nozzle cap may preferably be formed of a resin selected from the group consisting of polymethylpentene, polypropylene, polyethylene, polycarbonates, polyethylene terephthalate, and polycyclic olefins. Preferably, the plastic-made nozzle cap may also be formed of a light-transmitting resin having a visible light transmission of at least 70% at from 400 nm to 800 nm.

Although the plastic-made nozzle cap according to the present invention is made of a plastic, it can be firmly connected to the female Luer lock fitting of the plastic syringe and can also maintain high sealing performance even when continuously used over a validity term of a medicine.

### Brief Description of the Drawings

FIG. 1 is a schematic view of a plastic-made nozzle cap according to a first embodiment of the present invention.
FIG. 2 is a cross-sectional view of the plastic-made nozzle cap as seen in the direction of arrows II-II in FIG. 1.
FIG. 3 is a cross-sectional view of a plastic-made nozzle cap according to a second embodiment of the present invention.
FIG. 4 is a cross-sectional view of the plastic-made nozzle cap illustrated in FIG. 1 and combined with a syringe barrel.
FIG. 5 is a schematic view of the plastic-made nozzle cap according to the first embodiment of the present invention as combined with a syringe by way of example.

### Detailed Description of Preferred Embodiments

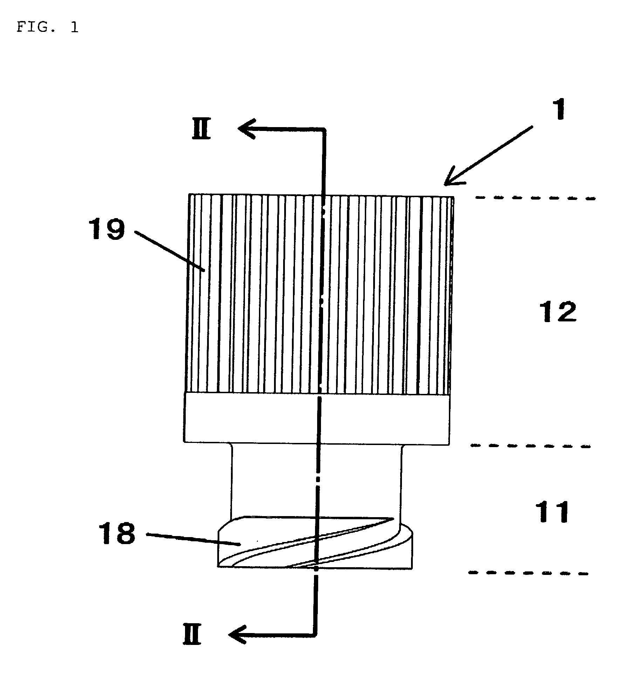

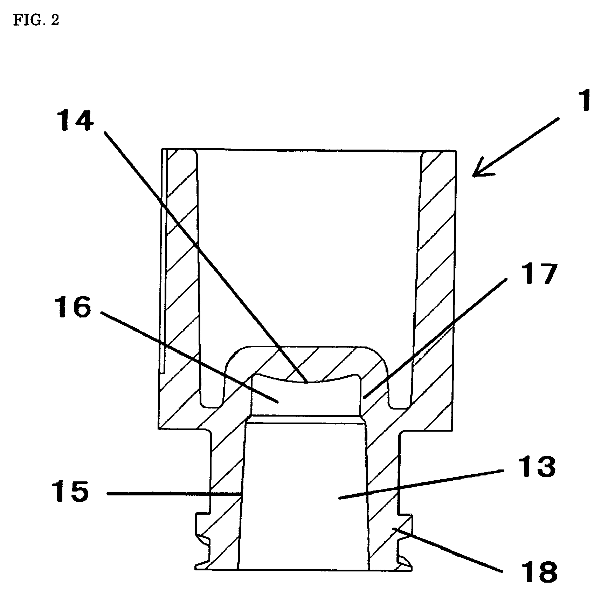

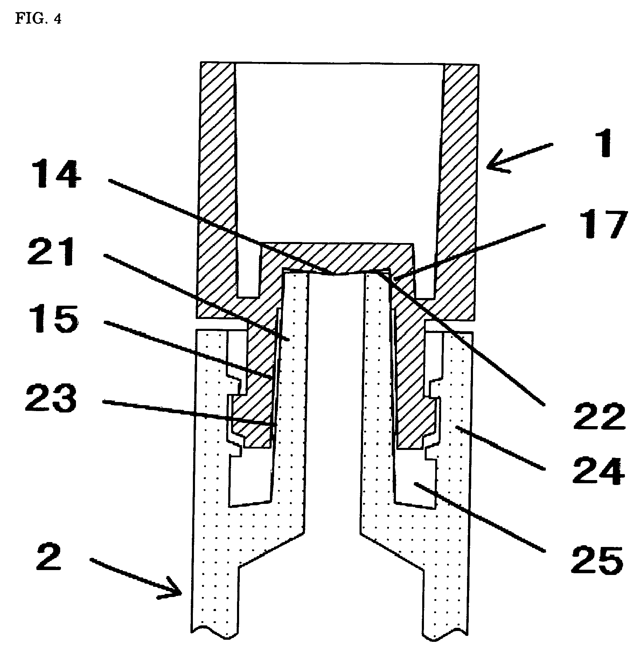

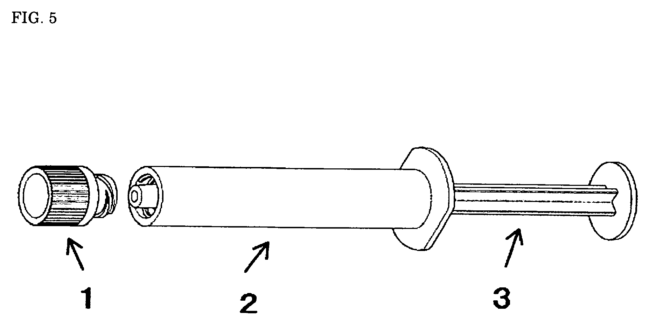

With reference to the drawings that illustrate preferred modes for practicing the present invention, the present invention will next be described in further detail. FIG. 1 is a schematic view of a plastic-made nozzle cap 1 according to a first embodiment of the present invention, and FIG. 2 is a cross-sectional view of the nozzle cap 1. FIG. 5 is a schematic view of the plastic-made nozzle cap illustrated in FIG. 1, and shows the plastic-made nozzle cap in threaded engagement with a female Luer lock fitting arranged at a tip portion of a syringe barrel, and FIG. 4 is a cross-sectional view illustrating the nozzle cap according to the first embodiment in threaded engagement with the female Luer lock fitting arranged at the tip portion of the syringe barrel.

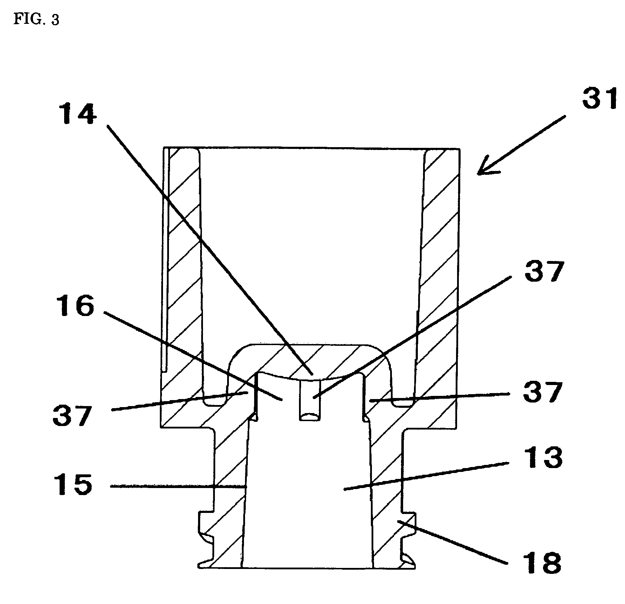

As illustrated in FIGS. 1 and 4, the plastic-made nozzle cap 1 according to the first embodiment is provided with a leg section 11, a cavity top wall portion 14, and a finger knob 12 for the attachment/detachment of the nozzle cap 1. The leg section 11 is structured for connection with the female Luer lock fitting 24 formed face-to-face with an outer circumferential wall 23 of a syringe nozzle 21. The cavity top wall portion 14 is provided with a downwardly-convex, curved surface for being brought into contact with a tip 22 of the syringe nozzle 21 to maintain sealing performance. The finger knob 12 is arranged on an upper side of the leg section 11, and extends above the female Luer lock fitting 24 in a state that the syringe barrel 2 and the nozzle cap 1 have been connected together. As illustrated in FIG. 2, a ring-shaped guide 17 may preferably be formed on an end part 16 of a cavity side wall 15, said end part 16 being located adjacent the cavity top wall portion 14. FIG. 3 is a cross-sectional view of a plastic-made nozzle cap 31 according to a second embodiment of the present invention, which has a guide different in shape from the ring shape depicted in FIG. 2.

As illustrated in FIG. 2, the leg section 11 has a cavity 13 into which the tip 22 of the syringe nozzle 21 can be inserted, and the cavity top wall portion 14 is formed at a top wall of the cavity 13. The cavity top wall portion 14 is provided with the downwardly-convex, curved surface, which has a radius of curvature ranging preferably from 0.6 to 12.0 mm, more preferably from 1.3 to 8.1 mm. A radius of curvature of smaller than 0.6 mm is not preferred, because such an excessively small radius of curvature makes the cavity top wall portion 14 substantially enter an end opening of the syringe nozzle 21 and cannot deny a potential problem that the cavity top wall portion 14 may be broken to produce foreign matter upon attachment or detachment of the nozzle cap 1. A radius of curvature of greater than 12.0 mm, on the other hand, is not preferred either, because such an excessively large radius of curvature makes substantially planar the surface at which the cavity top wall portion 14 is brought into contact with the tip 22 of the syringe nozzle 21, and leads to a higher risk that leakage may take place even with a slightest dimensional error of the tip 22 of the syringe nozzle 21. It has been confirmed that, when the radius of curvature is in the range of from 1.3 to 8.1 mm, the curved surface of the cavity top wall portion 14 is assured to come into contact with an inner circumferential edge of the end opening and can maintain sealing performance more stably provided that the inner diameter of the end opening of the syringe nozzle 21 is in a range of from 1.0 to 2.5 mm.

It is to be noted that for one provided at a cavity top wall portion thereof with a smaller radius of curvature than a half of the diameter of an end opening of a syringe nozzle of the associated syringe barrel, the cavity top wall portion is centrally provided with a hemispherical projection (not illustrated) having a radius of curvature equal to the half of the diameter such that the hemispherical projection can be fitted in the end opening of the syringe nozzle. For the formation of the entire cavity top wall portion in a curved shape without arrangement of any projection on the cavity top wall portion, it is, therefore, necessary to set the radius of curvature of the cavity top wall portion equal to or greater than the half of the diameter of the cavity top wall portion. As will be described subsequently herein, the diameter of the cavity top wall portion is set at approx. 3. 9 mm in the second embodiment of the present invention. In the second embodiment, the most preferred radius of curvature of the cavity top wall portion is, therefore, in a range of from 1.95 to 8.1 mm. On the other hand, the projection, which is arranged on the cavity top wall portion and is fit in the end opening of the syringe nozzle to assure sealing performance as disclosed in WO-A-2008-59863 and JP-A-2007-507308 cited above, is not practically suited to the present invention, because there is the potential problem that in the nozzle cap according to the present invention, such a projection may be broken to produce foreign matter as mentioned above. Such a projection is certainly an effective means for the assurance of sealing performance when the nozzle cap is produced with a high-flexibility material such as a rubber-based material. According to a study by the present inventor, however, such a projection is not effective for a plastic-made nozzle cap, but on the contrary, involves a potential problem that it may be broken to produce foreign matter, that is, a situation which must be absolutely avoided in an application to a medicine may arise. Such a situation does not arise provided that the diameter of the projection is sufficiently smaller than the diameter of the end opening of the syringe nozzle. In this case, however, the projection obviously does not contribute to the assurance and maintenance of sealing performance.

In the cross-sectional view of FIG. 4, the plastic-made nozzle cap 1 illustrated in FIG. 1 is in threaded engagement with the female Luer lock fitting 24 arranged at the tip portion of the syringe barrel 2. As seen in FIG. 4, it is not absolutely necessary for the cavity side wall 15 to be in contact with the outer circumferential wall 23 of the syringe nozzle 21. On the contrary, it is rather preferred to keep them out of contact for the purpose of protecting the outer circumferential wall 23 of the syringe nozzle 21 from scratches. Preferably, however, the ring-shaped guide 17 may be arranged on the end part 16 of the cavity side wall 15, said end part 16 being located adjacent the cavity top wall portion 14, such that the ring-shaped guide 17 can be brought into contact with an end part and its vicinity area of the outer circumferential wall 23 of the syringe nozzle 21 to effect an appropriate positional adjustment between the nozzle cap 1 and the syringe nozzle 21. As illustrated in FIG. 2, the ring-shaped guide 17 is formed surrounding, over a predetermined width, the entire circumference of the end part and its vicinity area of the outer circumferential wall 23 of the syringe nozzle 21. The ring-shaped guide 17 may be set preferably at from 0.3 to 1.0 mm or so in the height from the cavity side wall 15 and at from 0.5 to 3.0 mm or so in the width from the cavity top wall portion 14. The height of the ring-shaped guide 17 may conform to the dimension of the clearance between the cavity side wall 15 and the outer circumferential wall 23 of the syringe nozzle 21 in many instances.

It is also a preferred embodiment to set, as a uniform diameter, the inner diameter at the ring-shaped guide 17. As specified in the international standard (ISO 594/1 or ISO 594/2), the outer circumferential wall 23 of the syringe nozzle 21 is tapered such that its diameter progressively decreases toward the tip 22 of the syringe nozzle 21. By bringing the tapered outer circumferential wall 23 of the syringe nozzle 21 and the ring-shaped guide 17 of the untapered uniform diameter into close contact with each other, the position of the cavity top wall portion 14 and that of the tip 22 of the syringe nozzle 21 can be specified more accurately.

FIG. 3 illustrates the plastic-made nozzle cap 31 according to the second embodiment of the present invention, in which semicircular column-shaped protrusions (which may hereinafter be called "protrusion-shaped guides) 37 of approx. 0.4 mm in height, approx. 1.5 mm in width and approx. 0.4 mm in thickness are formed at positions where the end part 16 of the cavity side wall 15 is divided along an entire circumference thereof into 4 equal sections. In this second embodiment, the cavity side wall 15 is formed with a taper of approx. 6 degrees with respect to an axial direction of the plastic-made nozzle cap 31 such that the cavity side wall 15 progressively decreases in diameter toward the cavity top wall portion 14 in conformity with the shape of the syringe nozzle 21 (see FIG. 4). The protrusion-shaped guides 37 are formed on the cavity side wall 15 such that their inscribed circle has a uniform diameter of approx. 3.9 mm. Such protrusion-shaped guides may also be formed at positions where the end part 16 of the cavity side wall 15 is divided along the entire circumference thereof into 3 or 5 equal sections. The cross-sectional shape of each protrusion-shaped guide 37 is not limited to the semicircular column shape, but may be in a triangular column shape, square column shape or the like. These protrusion-shaped guides 37 may be set preferably at from 0.3 to 1.0 mm or so in the height from the cavity side wall 15 and at from 0.5 to 3.0 mm or so in the width from the cavity top wall portion 14. The height of each ring-shaped guide 37 may conform to the dimension of the clearance between the cavity side wall 15 and the outer circumferential wall 23 of the syringe nozzle 21 in many instances. The protrusion-shaped guides 37 are not required to be uniform in height, width and thickness. Further, these protrusion-shaped guides 37 may be suitably designed to guide the tip 22 (see FIG. 4) of the syringe nozzle 21 toward a central part of the cavity top wall portion 14 and to hold the syringe nozzle 21 in place.

The dimensions of the cavity 13 are set to permit the insertion of the syringe nozzle 21 (see FIG. 4) conforming to the international standard (ISO 594/1 or ISO 594/2) although they may vary depending on the connection mechanism of the leg section 11. The cavity 13 may be designed preferably to have a depth of from 5 to 8 mm or so, and at an opening located on a side farthest from the cavity top wall portion 14, a diameter of from 3.5 to 5 mm or so. Further, the cavity 13 may preferably be provided in its entirety with the same taper (approx. 6 degrees) or a taper of a similar angle as that formed at the syringe nozzle 21. The inner diameter at the ring-shaped guide 17 or protrusion-shaped guides 37 may preferably be the same as or a little greater than the outer diameter at the tip 22 of the syringe nozzle 21, that is, from 3.0 to 4.5 mm or so, and upwardly toward the tip 22 of the syringe nozzle 21, may be uniform or may progressively decrease with the same angle (approx. 6 degrees) or a similar angle as the taper of the syringe nozzle 21.

The connection mechanism formed on an outer side wall of the leg section 11 for connection with the female Luer lock fitting 24 of the syringe barrel 2 may be constructed as desired insofar as it can firmly connect to internal threads 25 formed at the female Luer lock fitting 24. Examples of the connection mechanism include external threads 18 threadedly engageable with the internal threads 25 of the female Luer lock fitting 24 as illustrated by way of example in FIGS. 2 and 3, and projections. Such a connection mechanism may be molded integrally with the nozzle cap 1 (31), or may be molded as a discrete member from the nozzle cap 1 (31) and may then be combined with the nozzle cap 1(31). The connection mechanism is a part that will remain subjected to a load until the prefilled syringe is used, and therefore, is required to have sufficient strength and deformation resistance. Leading flanks of the external threads 18 and groove flanks of the internal threads 25, said groove flanks being located face-to-face with the leading flanks, respectively, in other words, the flanks of the external threads 18 and the corresponding flanks of the internal threads 25, said former flanks and latter flanks being in contact with each other, respectively, when the nozzle cap 1(31) and the syringe barrel 2 are in threaded engagement with each other, may be formed preferably at right angles to the axial direction of the syringe barrel 2 as in the first embodiment illustrated in FIGS. 2 and 4, because such a design is resistant to thread loosening.

The finger knob 12 for attachment/detachment (see FIG. 1) is an extending section, which is generally molded on an upper side of and in integration with the leg section 11 and is adapted to perform the attachment or detachment of the nozzle cap 1(31). The finger knob 12 for attachment/detachment may be constructed as desired insofar as it permits easy attachment and detachment of the nozzle cap 1 (31). As illustrated by way of example in FIG. 1, the finger knob 12 for attachment/detachment may preferably be in the form of a cylinder with flutes 19 formed as an anti-slip for fingers on an outer circumference thereof.

As the material of the plastic-made nozzle cap according to the present invention, a light-transmitting resin having a visible light transmission of at least 70% at from 400 nm to 800 nm may be used preferably. Suitably usable examples include polymethylpentene, polypropylene, polyethylene, polycarbonates, polyethylene terephthalate, polycyclic olefins, and the like.

Especially from the standpoints of elution from the material and effects on stress relaxation, components other than polymers, such as flexibilizers, rubbers and elastomers, may desirably amount to preferably 10% or less, more preferably 5% or less, most preferably 3% or less. The inclusion of such a third component tends to impair the transparency of the material itself.

FIG. 5 is a schematic view of the plastic-made nozzle cap 1 according to the first embodiment of the present invention as combined with a syringe by way of example. The nozzle cap 1 is used with the syringe in which the female Luer lock fitting 24 is formed surrounding the syringe nozzle 21. Described specifically, the nozzle cap 1 is used in combination with the syringe barrel 2 and a plunger rod 3.

The material of the syringe for use in the present invention is need to be a plastic that can provide the tip 22 of the syringe nozzle 21 with high dimensional accuracy. Glass-made syringes the dimensional specifications of which are strictly controlled may also be used to some extent. These glass-made syringes are, however, accompanied by a problem in that their production yield is low, their product quality can be hardly guaranteed, and loosening tends to occur at the connected parts as glass has low friction resistance with plastic components. As the material of the syringe, any material can be used insofar as it is commonly used as a material in syringes. Polycyclic olefin materials are particularly preferred from the standpoints of dimensional accuracy, heat resistance, chemical stability, transparency and the like.

### Examples 1 and 2

Using polymethylpentene ("DX845", trade name, product of Mitsui Chemicals, Inc.), nozzle caps of the structure shown in FIG. 2 were produced by injection molding. They were provided as the nozzle caps of Example 1. They had the following dimensions - the outer diameter of a finger knob for attachment/detachment: approx. 10 mm, the length of the finger knob for attachment/detachment: approx. 10 mm, the diameter of an outer circumference of a leg section: approx. 7 mm, the length of the outer circumference of the leg section: approx. 5 mm, the diameter of a cavity on the side of its opening: approx. 4.8 mm, the diameter of the cavity on the side of a cavity top wall: approx. 4.3 mm, the depth of the cavity: approx. 7 mm, the inner diameter of a ring-shaped guide: 3.9 mm (uniform diameter), the length of the ring-shaped guide: 1.5 mm, and the radius of curvature of a cavity top wall portion: 5. 0 mm. In a similar manner as in Example 1 except for the omission of the ring-shaped guide, nozzle caps were produced and provided as the nozzle caps of Example 2. Those nozzle caps were combined with syringe barrels ("CZ SYRINGE BARREL 1LL-S", trade name, product of Daikyo Seiko, Ltd.) and pistons ("1 ML PISTON NF-2", trade name, product of Daikyo Seiko, Ltd.), respectively, and the following evaluations were performed. In each evaluation, a test was performed on samples which had been allowed to stand for 6 months, in addition to an immediate test.

### Example 3

In a similar manner as in Example 1 except for the replacement of the ring-shaped guide in each nozzle cap by the protrusion-shaped guides depicted in FIG. 3, nozzle caps were produced and provided as the nozzle caps of Example 3. The dimensions and the like of the protrusion-shaped guides were set as in the second embodiment.

### Examples 4 to 7

In a similar manner as in Example 2 except that the radius of curvature of the cavity top wall portion of each nozzle cap was changed to 0.6, 1.3, 8.1 and 12.0 mm, respectively, nozzle caps were produced. Those nozzle caps were provided as the nozzle caps of Examples 4 to 7, respectively.

### Comparative Examples 1 to 3

In a similar manner as in Example 1 except that the radius of curvature of the cavity top wall portion of each nozzle cap was changed to 0 (planar), 0.5 and 12.5 mm, respectively, nozzle caps were produced. Those nozzle caps were provided as the nozzle caps of Comparative Examples 1 to 3, respectively.

### Referential Examples 1 and 2

In a similar manner as in Examples 1 and 3 except for the use of a material obtained by blending an SEBS elastomer ("TUFTEC H1221", trade name, product of Asahi Kasei Chemicals Corporation; 20 parts by weight) with the polymethylpentene (100 parts by weight), nozzle caps were produced. Those nozzle caps were provided as the nozzle caps of Referential Examples 1 and 2, respectively.

### Evaluation

The above-obtained nozzle caps of the Examples, Comparative Examples and Referential Examples were fitted on the nozzles of the plastic syringes, respectively, and were evaluated for air tightness as will be described below.

### Air Tightness Test

With reference to the leakage test as defined in the "Standards for Sterile Injection Syringes", a notification from the Director of the Safety Bureau, the Ministry of Health and Welfare (now, the Pharmaceutical and Food Safety Bureau, the Ministry of Health, Labor and Welfare) of Japan, 10 nozzle caps were used per each of the Examples, Comparative Examples and Referential Examples, and a confirmation test was performed for sealing performance by the following procedure.

Into each syringe barrel, the piston was inserted to a position where the internal volume of the syringe was 1 mL. Each nozzle cap was threadedly applied by hand tightening to the syringe until the nozzle cap became no longer turnable.

Upon elapse of a predetermined period of time, the syringe was fixed with the nozzle directed downward in a water-filled container while being submerged to a half or so of the syringe barrel. A plunger rod ("PLUNGER 1-2", trade name, manufactured by Daikyo Seiko, Ltd.) was threadedly connected to the piston. Using a precision universal tester ("AUTOGRAPH AG-5kNIS MS", trade name, manufactured by Shimadzu Corporation; the rated load of mounted load cell: 100 N), the plunger rod was pushed to a position where the volume of internal air was 0.4 mL. The push rate was set at 100 mm/min.

In the pushed state, the plunger rod was held in place for 15 seconds, during which the threadedly engaged parts of the syringe and nozzle were visually observed to confirm any leakage of air. The number of test samples was set at 10. Concerning the nozzle caps of the Examples, Comparative Examples and Referential Examples, the numbers of nozzle caps on which leakage of air was observed are presented in Tables 1 and 2.

### Heated Air-tightness Test

Ten (10) nozzle caps were used per each of the Examples, Comparative Examples and Referential Examples, and a confirmation test was performed for heated air-tightness by the below-described procedure. In the test, heated air-tightness was evaluated based on the number of nozzle caps on which leakage of air was observed. As an acceptability criterion, it was set that no leakage should be observed on two out of 10 samples (2/10) or more.

Into each syringe barrel, the piston was inserted to a position where the internal volume of the syringe was 1 mL. A 0.1% solution of methylene blue (1 mL) was subsequently injected into the syringe barrel through the opening at the tip of the nozzle. Each nozzle cap was threadedly applied by hand tightening to the syringe until the nozzle cap became no longer turnable.

Upon elapse of a predetermined period of time, the syringe barrel was lightly flicked with the nozzle directed downward to eliminate air inside the nozzle. The syringe was then fixed with the nozzle directed downward in a water-filled container while being submerged to a half or so of the syringe barrel.

The syringe was placed, together with the container, in a retort sterilizer ("LM-42MII", trade name, manufactured by Hirayama Manufacturing Corporation), and was heated at 121°C for 30 minutes. After completion of the heating, the container was taken out of the retort sterilizer, and the water in the container was visually observed for coloration to make a determination as to leakage. The number of test samples was set at 10. Concerning the nozzle caps of the Examples, Comparative Examples and Referential Examples, the numbers of nozzle caps on which leakage of air was observed are presented in Tables 1 and 2.

**Table 1 Evaluation Results of Examples**

| | | Ex. 1 | Ex. 2 | Ex. 3 | Ex. 4 | Ex. 5 | Ex. 6 | Ex. 7 |
|---|---|---|---|---|---|---|---|---|
| Air tightness test | Immediate | 0/10 | 0/10 | 0/10 | 0/10 | 0/10 | 0/10 | 0/10 |
| | After 6 months | 0/10 | 0/10 | 0/10 | 0/10 | 0/10 | 0/10 | 0/10 |
| Heated air-tightness test | Immediate | 0/10 | 0/10 | 0/10 | 0/10 | 0/10 | 0/10 | 0/10 |
| | After 6 months | 0/10 | 1/10 | 0/10 | 1/10 | 0/10 | 0/10 | 1/10 |

**Table 2 Evaluation Results of Comparative Examples and Referential Examples**

| | | Comp. Ex. 1 | Comp. Ex. 2 | Comp. Ex. 3 | Ref. Ex. 1 | Ref. Ex. 2 |
|---|---|---|---|---|---|---|
| Air tightness test | Immediate | 2/10 | 1/10 | 0/10 | 0/10 | 0/10 |
| | After 6 months | 4/10 | 3/10* | 2/10 | 1/10 | 2/10 |
| Heated air-tightness test | Immediate | 5/10 | 3/10 | 1/10 | 0/10 | 0/10 |
| | After 6 months | 6/10 | 7/10* | 4/10 | 2/10 | 3/10 |

| | | | | | | |
|---|---|---|---|---|---|---|
| * Damage occurred at cavity top wall portion. | | | | | | |

As has been described above, the plastic-made nozzle caps of the Examples of the present invention were confirmed to show high air tightness even under severe conditions that the test was conducted over the long period of time under heat, to say nothing of in the initial stage. They have hence been found to act as plastic-made nozzle caps that can maintain high sealing performance over a long period of time when fitted on plastic syringes. From the test results of the plastic-made nozzle caps of the Comparative Examples, it has also been confirmed that the shape of a cavity top wall portion affects air tightness. It has also been confirmed that, even with the plastic-made nozzle caps of the Referential Examples in which the material was changed to the elastomer-containing material, no problem would arise at all about air tightness insofar as they are used for a short period of time. However, the results of the air tightness test over the long period of time under the severe conditions have indicated that the plastic-made nozzle caps of the Referential Examples are inferior to the plastic-made nozzle caps of the Examples and that for the maintenance of air tightness under such severe conditions, the material of nozzle caps needs to be selected suitably.

## Claims

1. A plastic-made nozzle cap (1;31) for a plastic syringe useful with a medicine, **characterized by** comprising a leg section (11) structured for connection with a female Luer lock fitting (24) formed face-to-face with an outer circumferential wall of a nozzle (21) of the syringe, a cavity top wall portion (14) provided with a downwardly-convex, curved surface having a radius of curvature ranging from 0.6 to 12.0 mm such that the cavity top wall portion can be brought into contact with a tip (22) of the nozzle of the syringe to maintain sealing performance, and a finger knob (12) for attachment/detachment of the nozzle cap, said finger knob being arranged on an upper side of the leg section such that the finger knob extends above the female Luer lock fitting in a state that the syringe and the nozzle cap have been connected together.

2. The plastic-made nozzle cap according to claim 1, further comprising threads (18) or a ridge arranged on an outer circumference of the leg section (11) for threaded engagement with the female Luer lock fitting (24) of the syringe.

3. The plastic-made nozzle cap according to claim 1 or 2, further comprising a guide formed for the nozzle (21) on an end part (16) of a cavity side wall (15), said end part (16) being located adjacent the cavity top wall portion (14).

4. The plastic-made nozzle cap according to claim 3, wherein the guide comprises a ring-shaped guide (17).

5. The plastic-made nozzle cap according to claim 3, wherein the guide comprises plural semicircular column-shaped protrusions (37) formed at positions where the end part (16) of the cavity side wall (15) is divided along an entire circumference thereof into from 3 to 5 equal sections.

6. The plastic-made nozzle cap according to any one of claims 1-5, wherein the plastic-made nozzle cap is formed of a resin selected from the group consisting of polymethylpentene, polypropylene, polyethylene, polycarbonates, polyethylene terephthalate, and polycyclic olefins.

7. The plastic-made nozzle cap according to any one of claims 1-6, wherein the plastic-made nozzle cap is formed of a light-transmitting resin having a visible light transmission of at least 70% at from 400 nm to 800 nm.
